(19) Europäisches Patentamt / European Patent Office / Office européen des brevets

(11) **EP 3 749 323 B1**

(12) # EUROPEAN PATENT SPECIFICATION

(45) Date of publication and mention of the grant of the patent:
**20.05.2026 Bulletin 2026/21**

(21) Application number: **19747026.3**

(22) Date of filing: **02.02.2019**

(51) International Patent Classification (IPC):
***A61K 31/407*** *(2006.01)* ***A61K 31/535*** *(2006.01)*
***A61P 35/00*** *(2006.01)* ***A61P 29/00*** *(2006.01)*
***A61P 19/02*** *(2006.01)* ***A61K 31/404*** *(2006.01)*
***A61K 31/423*** *(2006.01)* ***A61K 31/437*** *(2006.01)*
***A61K 31/519*** *(2006.01)* ***A61K 39/00*** *(2006.01)*
***A61K 45/06*** *(2006.01)*

(52) Cooperative Patent Classification (CPC):
(C-Sets available)
**A61K 31/407; A61K 31/404; A61K 31/423; A61K 31/437; A61K 31/519; A61K 45/06; A61P 19/02; A61P 29/00; A61P 35/00;**
A61K 2039/542 (Cont.)

(86) International application number:
**PCT/CN2019/074618**

(87) International publication number:
**WO 2019/149286 (08.08.2019 Gazette 2019/32)**

(54) **HETEROBICYCLIC CARBOXYLIC ACIDS FOR TREATING CANCER OR INFLAMMATORY DISEASES**

HETEROBICYCLISCHE CARBONSÄUREN ZUR BEHANDLUNG VON KREBS ODER ENTZÜNDLICHEN ERKRANKUNGEN

ACIDES CARBOXYLIQUES HÉTÉROBICYCLIQUES POUR TRAITER LE CANCER OU DES MALADIES INFLAMMATOIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.02.2018 PCT/CN2018/075198**

(43) Date of publication of application:
**16.12.2020 Bulletin 2020/51**

(73) Proprietors:
- **Shenzhen Ionova Life Science Co., Ltd.**
  **Shenzhen, Guangdong 518118 (CN)**
- **Foshan Ionova Biotherapeutics Co., Inc.**
  **Chancheng District**
  **Foshan**
  **Guangdong 528000 (CN)**

(72) Inventors:
- **ZHOU, Gang**
  **Shenzhen, Guangdong 518118 (CN)**
- **SUN, Yongkui**
  **Shenzhen, Guangdong 518118 (CN)**
- **WANG, Zhaoyin**
  **Nanjing, Jiangsu 210049 (CN)**

(74) Representative: **FRKelly**
**Waterways House**
**Grand Canal Quay**
**Dublin D02 PD39 (IE)**

(56) References cited:
**WO-A1-2010/019796**
**WO-A1-2017/014323**
**WO-A1-2017/066633**
**WO-A1-2017/066633**
**CN-A- 102 149 384**
**US-A- 5 750 471**
**US-A1- 2006 264 493**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **BOYD MICHAEL J ET AL: "A novel series of potent and selective EP4receptor ligands: Facile modulation of agonism and antagonism", BIOORGANIC & MEDICINAL CHEMISTRY LETTERS, vol. 21, no. 1, 28 October 2010 (2010-10-28), pages 484 - 487, XP029121223, ISSN: 0960-894X, DOI: 10.1016/ J.BMCL.2010.10.106**
- **COLUCCI JOHN ET AL: "Discovery of 4-{1-[({1-[4-(trifluoromethyl)benzyl]-1H-indol-7-yl}carbonyl)amino]cyclopropyl}benzoic acid (MF-766), a highly potent and selective EP4antagonist for treating inflammatory pain", BIOORGANIC & MEDICINAL CHEMISTRY LETTERS, vol. 20, no. 12, 18 April 2010 (2010-04-18), pages 3760 - 3763, XP029212954, ISSN: 0960-894X, DOI: 10.1016/ J.BMCL.2010.04.065**


(52) Cooperative Patent Classification (CPC): (Cont.)

C-Sets

**A61K 31/407, A61K 2300/00**

## Description

BACKGROUND OF THE INVENTION

Prostaglandins are mediators of pain, fever and other symptoms associated with inflammation. Especially prostaglandin $E_2$ ($PGE_2$) is the predominant eicosanoid detected in inflammation conditions. In addition, it is also involved in various physiological and/or pathological conditions and such as hyperalgesia, uterine contraction, digestive peristalsis, awakeness, suppression of gastric acid secretion, blood pressure, platelet function, bone metabolism, angiogenesis or the like. Four $PGE_2$ receptor subtypes ($EP_1$, $EP_2$, $EP_3$ and $EP_4$) displaying different pharmacological properties have been cloned. $EP_4$ subtype, a Gs-coupled receptor, stimulates cAMP production and is distributed in a wide variety of tissue suggesting major role in $PGE_2$-mediated biological events. Patent application publications WO 96/06822, WO 96/11902, EP 752421-A1, WO03/16254, WO05/021508, and WO 07/121578 disclose compounds as being useful in the treatment of prostaglandin mediated diseases. Three review articles describe the characterization and therapeutic relevance of the prostanoid receptors as well as the most commonly used selective agonists and antagonists: Eicosanoids: From Biotechnology to Therapeutic Applications, Folco, Samuelsson, Maclouf, and Velo eds, Plenum Press, New York, 1996, chap. 14, 137-154; Journal of Lipid Mediators and Cell Signaling, 1996, 14, 83-87; and Prostaglandins and Other Lipid Mediators, 2002, 69, 557-573.

PGE2 favors a pro-inflammatory immune response; however, PGE2 has been implicated as an important constituent in the immunosuppressive environment created by many solid tumors (Whiteside, Expert Opinion in Biological Therapy, 2010. 10, 1019-1035), sustained levels in tumor microenvironment promote the accumulation and enhance the activity of multiple immunosuppressor cells, including tumor associated macrophages (TAM), Treg cells, and myeloid-derived suppressor cells (MDSCs), and consequently promote tumor immune escape. Accumulating evidence has demonstrated that elevated cAMP levels through EP4 are the primary signal leading to immunosuppression in immune cells (Yokoyama U et al., Pharmacol. Rev., 2013, 65:1010-1052). Studies have also shown that antagonists of prostaglandin E receptor 4 (EP4) can effectively induce inflammation (Chen et al., British J Pharmacol., 2010, 160, 292-310) by blocking of prostaglandin E2 (PGE2) signaling through the interaction of PGE2 with prostaglandin E receptor 4 subtype. Knockout of EP4 in mice showed delayed tumorigenesis compared to wild-type animals in the background of $APC_{min}$ mutation, indicating a tumor-promoting activity of PGE2-EP4 signaling in host immune cells (Mutoh M et al., Cancer Res., 2002, 62:28-32). Consistently, selective EP4 receptor antagonists have been shown to slow tumor progression and tumor metastasis in various preclinical tumor models without affecting the cancer cell proliferation in vitro (Yang et al., Cancer Res., 2006, 66:9665-9672; Mao Y et al., Clin. Cancer Res., 2014, 20:4096-4106).

Based on such research, antagonists of the EP4 subtype of $PGE_2$ receptors would therefore have therapeutic value in the treatment of diseases or conditions mediated by the EP4 receptor, such as cancer and inflammatory diseases or conditions (e.g., acute and chronic pain, osteoarthritis, rheumatoid arthritis).

SUMMARY OF THE INVENTION

Provided is compound for use in a method for treating a cancer or an inflammatory disease, the use comprising administering to a subject in need thereof a compound as below or a pharmaceutically acceptable salt thereof,

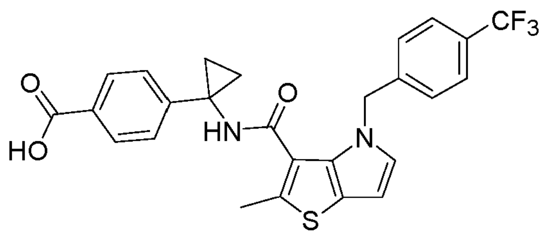

**INV-1120**

or

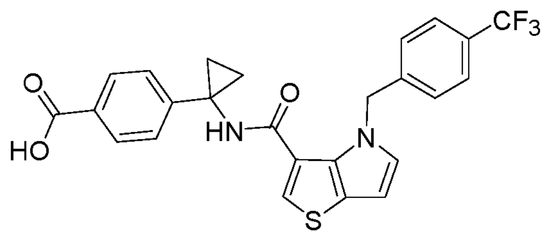

**INV-1121**

.

These compounds are also referred to as the "compounds of Formula I".

The compounds of the invention are antagonists of the EP4 receptor and are therefore useful in treating prostaglandin E2 mediated disease or conditions selected from cancer or an inflammatory disease. The EP4 antagonists described in this invention have an antagonistic action towards prostaglandin upon in vivo biotransformation and are thus useful in therapeutics, particularly for the treatment of a disorder or condition selected from cancer or an inflammatory disease.

Optionally, the cancer is breast cancer, endometrial cancer, cervix cancer, ovary cancer, lung cancer, head and

neck cancer, brain cancer, thyroid cancer, esophagus cancer, stomach cancer, colon and rectal cancer, liver cancer, pancreatic cancer, skin cancer, kidney cancer, bladder cancer, prostate cancer, testis cancer, bone cancer, Lymphoma, or blood cancer.

Optionally, the inflammatory disease is arthritis, acne vulgaris, asthma, autoimmune diseases, autoinflammatory diseases, Celiac disease, chronic prostatitis, colitis, diverticulitis, glomerulonephritis, hidradenitis suppurativa, hypersensitivities, inflammatory bowel diseases, interstitial cystitis, Mast Cell Activation Syndrome, mastocytosis, otitis, pelvic inflammatory disease, reperfusion injury, rheumatic fever, rheumatoid arthritis, rhinitis, sarcoidosis, or vasculitis.

Pharmaceutical compositions comprising the claimed compounds for the same use in treatments are also included within the scope of the invention.

The invention encompasses the compounds of Formula I for use in a method of treating an inflammatory disease susceptible to treatment with a non-steroidal anti-inflammatory agent comprising administering to a patient in need of such treatment of a non-toxic therapeutically effective amount of a compound of Formula I. Within this embodiment is encompassed the above method wherein the patient is also at risk of a thrombotic cardiovascular event and/or GI ulceration/bleeding.

Another embodiment of the disclosure (not claimed) encompasses the compounds of Formula I for use in method of treating prostaglandin E2 mediated diseases advantageously treated by an active agent that selectively antagonizes EP4 in preference to COX-1/COX-2 inhibition comprising administering to a patient in need of such treatment of a non-toxic therapeutically effective amount of a compound of Formula I. Within this embodiment is encompassed the above method wherein the patient is also at risk of a thrombotic cardiovascular event.

By way of example and without being limiting, the compounds described herein may be provided for use for cancer immune therapy targeting host immunosuppressive cells in the tumor microenvironment that can be of either myeloid or lymphoid lineage. In an embodiment, the compounds described herein may be used to treat patients with a variety of tumor types, including those that harbor high levels of myeloid infiltrate. Such levels of myeloid infiltrate may be identified, for example, based on the Cancer Genome Atlas (TCGA) and other sources. Such tumor types may also be identified based on protein or genetic (e.g., mRNA) expression analysis.

Tumor types may include but are not limited to pancreatic adenocarcinoma, renal Clear cell carcinoma, squamous cell carcinoma of head and neck (SCCHN), non-small cell lung cancer (NSCLC), colorectal cancer (CRC), hepatocellular carcinoma (HCC), serous epithelial ovarian cancer, cervical cancer, transitional cell bladder cancer, skin cancer, glioblastomas, kidney cancer, prostate cancer. pancreatic cancer, and triple-negative breast cancer (TNBC). In more particular aspects of the invention, provided are compounds for use in methods of treating cancer and/or generating a memory immune response comprising administering a compound below or a pharmaceutically acceptable salt thereof:

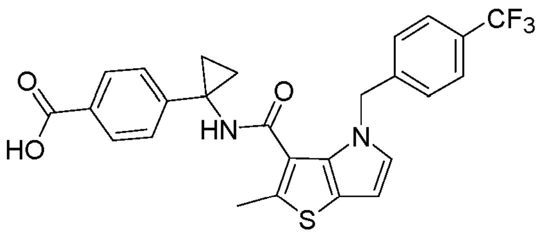

**INV-1120**

or

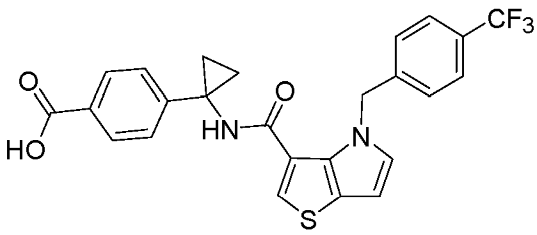

**INV-1121**

The compounds of the present invention are useful for treating or preventing a neoplasia in a subject in need of such treatment or prevention. The treatment includes partial or total inhibition of the neoplasia growth, spreading or metastasis, as well as partial or total destruction of the neoplastic cells. The term "prevention" includes either preventing the onset of clinically evident neoplasia altogether or preventing the onset of a preclinically evident stage of neoplasia in individuals at risk. Also intended to be encompassed by this definition is the prevention of initiation for malignant cells or to arrest or reverse the progression of premalignant cells to malignant cells. This includes prophylactic treatment of those at risk of developing the neoplasia. The term "subject" for purposes of treatment includes any human or mammal subject who has any one of the known neoplasias, and preferably is a human subject. For the claimed compounds for use in methods of prevention (not claimed), the subject is any human or animal subject, and preferably is a human subject who is at risk for obtaining a neoplasia. The subject may be at risk due to exposure to carcinogenic agents, being genetically predisposed to have the neoplasia, and the like.

The anti-tumor activities of various combinations of an EP4 antagonist with: radiation; antibodies to cytotoxic t-1ymphocyte antigen 4 (anti-CTLA4); antibodies to programmed death ligand 1 (anti-PDL1); antibodies to programmed cell death protein 1 (anti-PD1); and antimetabolites have been examined. The results from this examination have indicated improved and/or synergistic anti-tumor activities by the combination of the EP4 antagonist with the other therapies as

compared to single agent treatment alone, and in some embodiments this may result in a memory immune response against the tumor, even as against a different cancer. Thus, in one aspect of the invention, provided is are the claimed compounds for use in a method of treating cancer in a subject in need thereof comprising administering an EP4 antagonist selected form the claimed compounds in combination with a therapy selected from the group consisting of radiation therapy, antibody therapy and anti-metabolite chemotherapy. In a more particular aspect of the invention, the antibody therapy is selected from the group consisting of CTLA4 antibody therapy, PDL1 antibody therapy, and PD1 antibody therapy. In some embodiments, the cancer is metastatic cancer. In another aspect of the invention, provided is a method of generating a memory immune response in a subject in need thereof comprising administering an amount of an EP4 antagonist in combination with a therapy selected from the group consisting of radiation therapy, antibody therapy and anti-metabolite chemotherapy. In another more particular aspect of the invention, the antibody therapy is selected from the group consisting of CTLA4 antibody therapy, PDL1 antibody therapy and PD1 antibody therapy. The invention also encompasses the claimed compounds for use in a method of treating cancer with an effective amount of a compound of the present invention or using a combination of an effective amount of a compound of the present invention with an effective amount of radiation; antibodies to cytotoxic t-lymphocyte antigen 4 (anti-CTLA4); antibodies to programmed death ligand 1 (anti-PDLI); antibodies to programmed cell death protein 1 (anti-PD1); indoleamine-2,3-dioxygenase (IDO) inhibitors; tryptophan-2,3-dioxygenase (TDO) inhibitors; and antimetabolites. These antibodies can be selected from, but not limited to, MDX-010 (ipilimumab, Bristol-Myers Squibb), CP-675,206 (tremelimumab, Pfizer), MPDL3280A (Roche), MDX-1106 (nivolumab, Bristol- Myers Squibb), labrolizumab (Merck), and pembrolizumab (KEYTRUDA®, Merck).

BRIEF DESCRIPTIONS OF THE DRAWINGS

Fig. 1 shows the inhibitory effect of Compound 1 (INV-1121) on tumor growth in mice.
Fig. 2 shows the inhibitory effects of different treatments with or without Compound 1 on tumor growth in mice.
Fig. 3 shows results of in vivo inhibitory effect on the growth of B16F10 melanoma.
Fig. 4 shows the in vivo inhibitory effect on the growth of Lewis Lung Cancer.
Fig. 5 shows the temperature change of the paws of mice administered with the tested compound.
Fig. 6 shows the swelling reduction of the paws of mice administered with the tested compound.

DETAILED DESCRIPTION OF THE INVENTION

**Definitions**

Abbreviations used herein have their conventional meaning within the chemical and biological arts.

An "EP4 antagonist" refers to a compound which inhibits or blocks the cellular signaling triggered by the interaction of PGE2 with the EP4 receptor. The compounds of Formula (1) as taught herein, consist of INV-1120 and INV-1121, which are described in PCT/US2009/0537482 and WO2010/019796.

The invention is limited to use in the treatment of cancer or an inflammatory disease. The term "treating a prostaglandin E2 mediated disease or condition" means treating or preventing any chronic disease or condition that is advantageously treated or prevented selective EP4 antagonists. The term includes the relief of pain, fever and inflammation of a variety of conditions including rheumatic fever, symptoms associated with influenza or other viral infections, common cold, low back pain, neck pain, dysmenorrhea, headache, migraine, toothache, sprains and strains, myositis, neuralgia, synovitis, arthritis, including rheumatoid arthritis, degenerative joint diseases (osteoarthritis), gout, ankylosing spondylitis, bursitis, burns, injuries, and pain and inflammation following surgical procedures. In addition, such a compound may inhibit cellular neoplastic transformations and metastatic tumor growth and hence can be used in the treatment and/or prevention of cancer.

The term "treatment," "treat," or "treating" refers to alleviating, inhibiting and/or reversing the progress of a cancer in a subject in need thereof. The term "treating" is inclusive of any indicia of success in the treatment or amelioration of the cancer, including any objective or subjective parameter such as abatement; remission; diminishing of symptoms or making the injury, pathology or condition more tolerable to the subject; delaying or slowing in the rate of progression, etc. Measurement of the treatment or amelioration may be based on, e.g., the results of a physical examination, a pathological test and/or a diagnostic test as known in the art. Treating may also refer to reducing the incidence or onset of a cancer, or a recurrence thereof (such as a lengthening in time of remission), as compared to that which would occur in the absence of the measure taken.

The term "neoplasia" includes both benign and cancerous tumors, growths and polyps. Thus, the compounds of the invention are useful for treating or preventing benign tumors, growths and polyps including squamous cell papilloma, basal cell tumor, transitional cell papilloma, adenoma, gastrinoma, cholangiocellular adenoma, hepatocellular adenoma,

renal tubular adenoma, oncocytoma, glomus tumor, melanocytic nevus, fibroma, myxoma, lipoma, leiomyoma, rhabdomyoma, benign teratoma, hemangioma, osteoma, chondroma and meningioma. The compounds of the invention are also useful for treating or preventing cancerous tumors, growths and polyps including squamous cell carcinoma, basal cell carcinoma, transitional cell carcinoma, adenocarcinoma, malignant gastrinoma, cholangiocelleular carcinoma, hepatocellular carcinoma, renal cell carcinoma, malignant melanoma, fibrosarcoma, myxosarcoma, liposarcoma, leimyosarcoma, rhabdomyosarcoma, malignant teratoma, hemangiosarcoma, Kaposi sarcoma, lymphangiosarcoma, ostreosarcoma, chondrosarcoma, malignant meningioma, non-Hodgkin lymphoma, Hodgkin lymphoma and leukemia. For purposes of this specification, "neoplasia" includes brain cancer, bone cancer, epithelial cell-derived neoplasia (epithelial carcinoma), basal cell carcinoma, adenocarcinoma, gastrointestinal cancer such as lip cancer, mouth cancer, esophogeal cancer, small bowel cancer and stomach cancer, colon cancer, rectal cancer, liver cancer, bladder cancer, pancreas cancer, ovary cancer, cervical cancer, lung cancer, breast cancer and skin cancer, such as squamus cell and basal cell cancers, prostate cancer, renal cell carcinoma, and other known cancers that affect epithelial, mesenchymal or blood cells throughout the body. The compounds of the invention are useful for treating or preventing any of the afore-mentioned cancers. The compounds of the invention are useful for treating or preventing benign and cancerous tumors, growths and polyps of the following cell types: squamous epithelium, basal cells, transitional epithelium, glandular epithelium, G cells, bile ducts epithelium, hepatocytes, tubules epithelium, melanocytes, fibrous connective tissue, cardiac skeleton, adipose tissue, smooth muscle, skeletal muscle, germ cells, blood vessels, lymphatic vessels, bone, cartilage, meninges, lymphoid cells and hematopoietic cells. The compounds can be used to treat subjects having adenomatous polyps, including those with familial adenomatous polyposis (FAP). Additionally, the compounds can be used to prevent polyps from forming in patients at risk of FAP. Preferably, the compounds of the invention are useful for treating or preventing the following cancers: colorectal, esophagus stomach, breast, head and neck, skin, lung, liver, gall bladder, pancreas, bladder, endometrium cervix, prostate, thyroid and brain.

"Cancer" as used herein may include cancers that are the result of genetically inherited mutations. Examples of such cancers include, but are not limited to, breast cancers, cancers which can be related to Li-Fraumeni syndrome, for example, childhood sarcomas, leukemias and brain cancers, cancers which can be related to Lynch syndrome, for example, colon cancers, bile duct cancers, brain cancers, endometrial cancers, kidney cancers, ovarian cancers, pancreatic cancers, small intestinal cancers, stomach cancers and ureter cancers, lung cancers, melanomas, prostate cancers, retinoblastoma, thyroid cancer and uterine cancers. Moreover, cancer can be the result of acquired mutations, for example, mutations resulting from diet, environment and/or lifestyle, or somatic mutations. Examples of such cancers may include, but are not limited to, adrenal cancer, adrenal cortex cancer, bladder cancer, brain cancer, primary brain cancer, glioma, glioblastoma, breast cancer, cervical cancer, colon cancer (non-limiting examples include colorectal carcinomas such as colon adenocarcinoma and colon adoma), endometrial cancer, epidermal cancer, esophageal cancer, gall bladder cancer, genitourinary cancer, head or neck cancer, kidney cancer, liver cancer, lung cancer (non-limiting examples include adenocarcinoma, small cell lung cancer and non-small cell lung cancer), lymphomas (non-limiting examples include B-cell lymphoma, T-cell lymphoma, Hodgkin's lymphoma, non-Hodgkin's lymphoma), melanoma, malignant melanoma, malignant carcinoid carcinoma, malignant pancreatic insulinoma, myeloma, multiple myeloma, ovarian cancer, pancreatic cancer (such as exocrine pancreatic carcinoma), prostate cancer, renal cell cancer, skin cancer, such as, in addition to others previously mentioned, squamous cell carcinoma, stomach cancer, testicular cancer, thyroid cancer, thyroid follicular cancer, Wilms' tumor, choriocarcinoma, mycosis fungoides, malignant hypercalcemia, cervical hyperplasia, leukemia, acute lymphocytic leukemia, chronic lymphocytic leukemia, hairy cell lymphoma, Burkett's lymphoma, acute myelogenous leukemia, chronic myelogenous leukemia, myelodysplastic syndrome, promyelocytic leukemia, chronic granulocytic leukemia, acute granulocytic leukemia, fibrosarcoma, habdomyosarcoma, astrocytoma, neuroblastoma, rhabdomyosarcoma, schwannoma, Kaposi's sarcoma, polycythemia vera, essential thrombocytosis, Hodgkin's disease, non-Hodgkin's lymphoma, soft-tissue sarcoma, osteogenic sarcoma, primary macroglobulinemia, seminoma, teratocarcinoma, osteosarcoma, xenoderoma pigmento, keratoctanthoma and retinoblastoma.

The cancer treated is selected from the group consisting of breast cancers, cervical cancers, colorectal cancers, endometrial cancers, glioblastomas, head and neck cancers, kidney cancers, liver cancers, lung cancers, medulloblastomas, ovarian cancers, pancreatic cancers, prostate cancers, skin cancers and urinary tract cancers.

The compound of Formula(I) is:

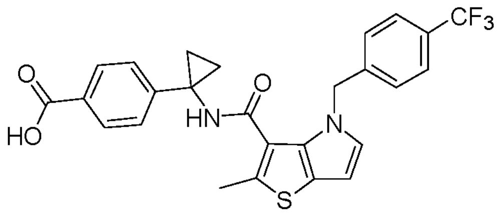

INV-1120

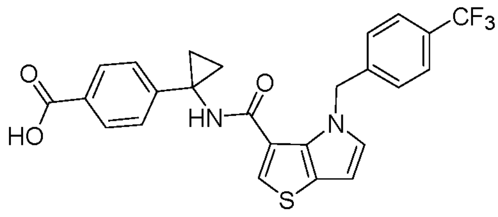

INV-1121

or a pharmaceutically acceptable salt thereof.

It will be appreciated that certain compounds of Formula I (or pharmaceutically acceptable salts thereof ) may exist in, and be isolated in, isomeric forms, including tautomeric forms, cis- or trans-isomers, as well as optically active, racemic, or diastereomeric forms. It is to be understood that the present invention encompasses a compound of Formula I in any of the tautomeric forms or as a mixture thereof; or as a mixture of diastereomers, as well as in the form of an individual diastereomers, and that the present invention encompasses a compound of Formula I as a mixture of enantiomers, as well as in the form of an individual enantiomer, any of which mixtures or form possesses antagonistic properties against EP4 receptor, it being well known in the art how to prepare or isolate particular forms and how to determine antagonistic properties against EP4 receptor by standard tests including those described herein below.

In addition, a compound of Formula I (or pharmaceutically acceptable salts thereof thereof) may exhibit polymorphism or may form a solvate with water or an organic solvent. The present invention also encompasses any such polymorphic form, any solvate or any mixture thereof.

As mentioned above, the invention includes a pharmaceutically acceptable salt of a compound of Formula I. A basic compound of this invention possesses one or more functional groups sufficiently basic to react with any of a number of inorganic and organic acids affording a physiologically acceptable counterion to form a pharmaceutically acceptable salt.

The invention also encompasses use of a compound of Formula I for the manufacture of a medicament for the treatment of a disease or condition that is mediated by the action of PGE2 at EP4 receptors.

Optical Isomers - Diastereomers - Geometric Isomers - Tautomers

Compounds of Formula I contain one or more asymmetric centers and can thus occur as racemates and racemic mixtures, single enantiomers, diastereomeric mixtures and individual diastereomers. The present invention is meant to comprehend all such isomeric forms of the compounds of Formula I to Formula Ig.

Some of the compounds described herein contain olefinic double bonds, and unless specified otherwise, are meant to include both E and Z geometric isomers.

Compounds described herein may exist with different points of attachment of hydrogen, referred to as tautomers. For example, a ketone and its enol form are known as keto-enol tautomers. The individual tautomers as well as mixtures thereof are encompassed with compounds of Formula I to Formula Ig.

Compounds of Formula I may be separated into diastereoisomeric pairs of enantiomers by, for example, fractional crystallization from a suitable solvent, such as MeOH or EtOAc or a mixture thereof. Enantiomers thus obtained may be separated into individual stereoisomers by conventional means, for example by use of an optically active amine as a resolving agent, or on a chiral HPLC column.

Alternatively, any enantiomer of a compound of Formula I may be obtained by stereospecific synthesis using optically pure starting materials or reagents of known configuration.

## Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc, and the like. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the

like.

When a compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like. Particularly preferred are citric, hydrobromic, hydrochloric, maleic, phosphoric, sulfuric, and tartaric acids.

It will be understood that, as used herein, references to the compounds of Formula I are meant to also include the pharmaceutically acceptable salts.

The term "crystal polymorphs", "polymorphs" or "crystal forms" means crystal structures in which a compound (or a salt or solvate thereof) can crystallize in different crystal packing arrangements, all of which have the same elemental composition. Different crystal forms usually have different X-ray diffraction patterns, infrared spectral, melting points, density hardness, crystal shape, optical and electrical properties, stability and solubility. Recrystallization solvent, rate of crystallization, storage temperature, and other factors may cause one crystal form to dominate. Crystal polymorphs of the compounds can be prepared by crystallization under different conditions. It is understood that the compounds of the present disclosure may exist in crystalline form, crystal form mixture, or anhydride or hydrate thereof.

Compounds of Formula I can also be used in combination with one or more chemotherapeutic agents such as an aromatase inhibitor, an antiestrogen, an anti-androgen (especially in the case of prostate cancer) or a gonadorelin agonist, a topoisomerase I inhibitor or a topoisomerase II inhibitor, a microtubule active agent, an alkylating agent, an antineoplastic antimetabolite or a platin compound, a compound targeting/decreasing a protein or lipid kinase activity or a protein or lipid phosphatase activity, a further anti-angiogenic compound or a compound which induces cell differentiation processes, a bradykinin I receptor or an angiotensin II antagonist, a cyclooxygenase inhibitor, a bisphosphonate, a rapamycin derivative such as everolimus, a heparanase inhibitor (prevents heparan sulphate degradation), e.g. PI 88, a biological response modifier, preferably a lymphokine or interferons, e.g. interferon if, an ubiquitination inhibitor, or an inhibitor which blocks anti-apoptotic pathways, an inhibitor of Ras oncogenic isoforms, e. g. H-Ras, K-Ras or N-Ras, or a farnesyl transferase inhibitor, e.g. L-744, 832 or DK8G557, a telomerase inhibitor, e.g. telomestatin, a protease inhibitor, a matrix metalloproteinase inhibitor, a methionine aminopeptidase inhibitor, e.g. bengamide or a derivative thereof, or a proteosome inhibitor, e.g. PS 341, histone deacetylase inhibitors, e.g. Vorinostat, MG0103 or MS275, or PTP 1B inhibitors.

Any references to "methods of treatment" are to be interpreted as "compounds of Formula I for use in treatment".The term "amount that is therapeutically effective to treat" is intended to mean that amount of a drug or pharmaceutical agent that will elicit the biological or medical response of a tissue, a system, animal or human that is being sought by a researcher, veterinarian, medical doctor or other clinician. The term also encompasses the amount of a pharmaceutical drug that will prevent or reduce the risk of occurrence of the biological or medical event that is sought to be prevented in a tissue, a system, animal or human by a researcher, veterinarian, medical doctor or other clinician. The EP4 antagonist may be administered at a dosage level up to conventional dosage levels. Suitable dosage levels will depend upon the effect of the chosen EP4 antagonist, but typically suitable levels will be about 0.001 to 100mg/kg per day, preferably 0.005 to 30 mg/kg per day, and especially 0.05 to 10 mg/kg per day. The compound may be administered on a regimen of once, twice or three times per day.

## Formulations

The present invention also provides a pharmaceutical composition for use in the above-described therapeutic methods. Pharmaceutical compositions of the present invention comprise a compound of Formula I as an active ingredient or a pharmaceutically acceptable salt, thereof, in an amount sufficient to antagonize EP4 receptor, and may also contain a pharmaceutically acceptable carrier and optionally other therapeutic ingredients. The term "pharmaceutically acceptable salts" refers to salts prepared from pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc, and the like. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like.

It will be understood that in the discussion of methods of treatment herein references to the compounds of Formula I are meant to also include the pharmaceutically acceptable salts.

Pharmaceutical compositions containing an active ingredient (i.e. a compound of Formula I) may be in a form

suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example, magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the technique described in U.S. Patent Nos. 4,256,108; 4,166,452; and 4,265,874, to form osmotic therapeutic tablets for controlled release.

## Combination Therapy

Compounds of Formula I may be used in combination with other drugs useful in the treatment of cancer or conditions for which compounds of Formula I are useful. Such other drugs may be administered, by a route and in an amount commonly used therefor, contemporaneously or sequentially with a compound of Formula I. When a compound of Formula I is used contemporaneously with one or more other drugs, a pharmaceutical composition containing such other drugs in addition to the compound of Formula I is preferred. Accordingly, the pharmaceutical compositions of the present invention include those that also contain one or more other active ingredients, in addition to a compound of Formula I. When compounds of the invention are used in combination with other therapeutic agents, the compounds may be administered either sequentially or simultaneously by any convenient route.

The disclosure thus provides, in a further non claimed aspect, a combination comprising a compound of Formula I or a pharmaceutically acceptable derivative or salt thereof together with a further therapeutic agent or agents.

The combinations referred to above may conveniently be presented for use in the form of a pharmaceutical formulation and thus pharmaceutical formulations comprising a combination as defined above together with a pharmaceutically acceptable carrier or excipient comprise a further non claimed aspect of the disclosure. The individual components of such combinations may be administered either sequentially or simultaneously in separate or combined pharmaceutical formulations.

In some embodiments of the present invention, provided are the claimed compounds for use in a method of inhibiting tumor growth or treating cancer wherein an EP4 antagonist is administered in combination with an additional therapy or agent useful for inhibiting tumor growth and/or treating cancer, i.e., a combination therapy. As used herein, the administration of two or more agents/therapies (inclusive of EP4 antagonists, radiation therapy, antibody therapy, anti-metabolite chemotherapy, or any combination thereof) "in combination" means that the therapies are administered closely enough in time that the administration of or presence of one alters the biological effects of the other. The therapies may be administered simultaneously (concurrently) or sequentially. Simultaneous administration may be carried out, e.g., by mixing two or more agents prior to administration, or by administering the agent/therapy at the same point in time but at different anatomic sites or using different routes of administration, or administered at times sufficiently c10se that the results observed are indistinguishable from those achieved when the agents/therapies are administered at the same point in time. For example, simultaneous administration of one or more agents with radiation may be carried out by administering the agent(s) at the same point in time as the radiation is applied, or at times sufficiently close that the results observed are indistinguishable from those achieved when the agent(s) and radiation are administered at the same point in time. Sequential administration may be carried out by administering the agents/therapies at different points in time, e.g., administering an agent at some point in time prior to or after administration of one or more other agents/therapies, such that the administration of the agents/therapies in combination enhances the therapeutic effect of cancer treatment. In some embodiments, an EP4 antagonist is administered at some point in time prior to the initial administration of radiation therapy, antibody therapy and/or anti-metabolite chemotherapy. Alternatively, the radiation therapy, antibody therapy and/or anti-metabolite chemotherapy may be administered at some point in time prior to the administration of the EP4 antagonist, and optionally, administered again at some point in time after the administration of the EP4 antagonist. In some embodiments, administration of the EP4 antagonist in combination with radiation therapy, antibody therapy and/or anti-metabolite chemotherapy results in an enhancement of said radiation therapy, antibody therapy and/or anti-metabolite chemotherapy such that, for example, a smaller dosage of the radiation, antibody therapy and/or anti-metabolite chemotherapy may be effective for treatment. In some embodiments of the invention, the treatment of cancer may comprise an abscopal effect and/or provide a memory immune response. An "abscopal" effect is a phenomenon in the treatment of a metastatic cancer in which localized treatment of a particular tumor or cancer with, for example, radiation therapy, results in the shrinking and

disappearance of non-localized disease, tumors or cancer, such as those resulting from metastasis that are distant from the site of localized treatment, thus leading to the disappearance of disease, tumors or cancer throughout the subject or patient. An abscopic effect differs from effects that may occur on tissues adjacent to the localized treatment, such as, for example, bystander effects that may result from radiation therapy. A "memory immune response" results when the provided treatment for cancer facilitates the adaptation of the immune system and the immune response of the subject or patient in its ability to slow, reduce or prevent the rectum or the recurrence, e.i., lengthening the time of remission, of the disease, tumor or cancer being treated in the subject or patient. In some embodiments, the memory immune response may slow, reduce or prevent the development of tumors or cancers that are different than the cancer being treated, e.g., through epitope spreading. The EP4 antagonist, antibody and/or anti-metabolite as used herein may be formulated for administration in a pharmaceutical carrier in accordance with known techniques. See, for example, Remington, The Science and Practice of Pharmacy (9th Ed. 1995). In the manufacture of a pharmaceutical formulation according to the invention, the active compound (including the physiologically acceptable salts thereof) is typically admixed with, inter alia, an acceptable carrier. The carrier must be acceptable in the sense of being compatible with any other ingredients in the formulation and must not be deleterious to the patient. The carrier may be a solid or a liquid, or both, and is preferably formulated with the compound as a unit-dose formulation, for example, a tablet, which may contain from 0.01 or 0.5% to 95% or 99% by weight of the active compound. One or more active compounds may be incorporated in the formulations of the invention, which may be prepared by any of the well-known techniques of pharmacy comprising admixing the components, optionally including one or more accessory ingredients and/or excipients. In some embodiments, any of the composition, carrier, accessory ingredient(s) excipient(s) and/or the' formulation(s) of the invention comprise ingredients that are from either natural or non-natural sources. In other embodiments, any component of the composition(s), carrier(s), accessory ingredient, excipient(s) and/or the formulation(s) of the invention may be provided in a sterile form. Non-limiting examples of a sterile carrier include endotoxin-free water or pyrogen-free water. The EP4 antagonist, antibody and/or anti-metabolite can be administered to subjects by any suitable route, including orally (inclusive of administration via the oral cavity and further including administration via an orogastric feeding tube) , intraperitoneally, parenterally, by inhalation spray, topically (i.e., both skin and mucosal surfaces, including airway surfaces), transdermally, rectally, nasally (including a nasogastric feeding tube) , sublingually, buccally, vaginally or via an implanted reservoir. The term "parenteral" as used herein includes subcutaneous, intramuscular, intradermal, intravenous, intra-articular, intra-synovial, intrastemal, intrathecal, intrahepatic, intralesional and intracranial injection or infusion techniques. In a particular embodiment, the EP4 antagonist, antibody and/or anti-metabolite is administered orally. In another particular embodiment, the EP4 antagonist, antibody and/or anti-metabolite is administered intravenously. In some embodiments, the amount of the EP4 antagonist, antibody and/or anti-metabolite that may be combined with the excipient materials to produce a composition in a single dosage form will vary depending upon the host treated, and the particular route of administration. In some embodiments, the EP4 antagonist, antibody and/or anti-metabolite is provided as part of a sterile composition/formulation comprising the EP4 antagonist, antibody and/or anti-metabolite and an acceptable carrier and/or excipient. In some embodiments, the EP4 antagonist is administered to the subject in an effective amount. An effective amount is generally 0.01 mg/kg to 500 mg/kg body weight per day. In some embodiments, the pharmaceutically acceptable compositions may be formulated so that a dosage of from 0.01 mg/kg to 200 mg/kg or from 0.01 mg/kg to 100 mg/kg body weight per day of the compound can be administered to a patient receiving these compositions (e. g., based on a 75 kg human, a dosage of from0.75 mg to 7.5 g or 15 g). In certain embodiments, the compositions of the present invention are formulated to provide a dosage of from 0.01 mg/kg to 70 mg/kg (e.g., based on a 75 kg human, a dosage of from 0.75 mg to 5.25 g). In some embodiments, the effective dose of the EP4 antagonist is from about 0.5 to about 250 mg/kg, 1 to about 250 mg/kg, from about 2 to about 200 mg/kg, from about 3 to about 120 mg/kg, from about 5 to about 250 mg/kg, from about 10 to about 200 mg/kg, or from about 20 to about 120 mg/kg. In some embodiments, effective dosages include about 0.5 mg/kg, 1 mg/kg, 2 mg/kg, 3 mg/kg, 4 mg/kg, 5 mg/kg, 6 mg/kg, 8 mg/kg, 10 mg/kg, 20 mg/kg, 25 mg/kg, 40 mg/kg, 50 mg/kg, 60mg/kg, 75 mg/kg, 100 mg/kg, 120 mg/kg, 150 mg/kg, 175 mg/kg, 200mg/kg, 225mgl/kg, 250mg/kg, and 300 mg/kg. Dosage forms can be in the form, e.g., of tablets or capsules, and the effective dose may be provided in one or more tablets, capsules or the like, and be provided once a day or throughout the day at intervals, e.g., of 4, 8 or 12 hours. Tablets or capsules, for example, could contain, e.g., 10, 25, 50, 75, 100, 150, 200, 250, 300, 350, 400, 450, 500, 600, 700, 800, 900, 1,000, 1,100, or 1,250 mg of compound. For example, administration to a human subject of the EP4 antagonist in some embodiments may comprise a daily dosage of the EP4 antagonist in the range of 100-1,250, 150-1,000,200-800, or 250-750 mg, which daily dosage can be administered either once a day in its entirely or factions of which are administered throughout the day in intervals. Liquid formulations can also be prepared so that any dosage may readily and conveniently be dispensed. The antibody, e.g., anti-CTLA4, anti-PDL1 or anti-PD1, will generally be mixed, prior to administration, with a non-toxic, pharmaceutically acceptable carrier substance (e.g., normal saline or phosphate-buffered saline), and may be administered using any medically appropriate procedure, for example, including but not limited to, intravenous or intraarterial administration, and injection into the cerebrospinal fluid. In certain cases, intraperitoneal intradermal, intracavity, intrathecal or direct administration to tumor or to an artery supplying the tumor may be advantageous. In some embodiments, the effective dose of the antibody is from about 5 to about 250 mg/kg, from about 10 to about 200 mg/kg, or from about 20 to about 120 mg/kg. In some embodiments, effective

dosages include 5 mg/kg, 10 mg/kg, 20 mg/kg, 25 mg/kg, 40 mg/kg, 50 mg/kg, 60mg/kg, 75 mg/kg, 100 mg/kg, 120 mg/kg, 150 mg/kg, 175 mg/kg, 200 mg/kg, 225 mg/kg, 250 mg/kg, and 300 mg/kg. Dosage forms can be in the form, e.g., of tablets or capsules, and the effective dose may be provided in one or more tablets, capsules or the like, and be provided once a day or throughout the day at intervals, e.g., of 4, 8 or 12 hours. Tablets or capsules, for example, could contain, e.g., 10, 25, 50, 75, 100, 150,200,250,300,350,400,450, 500, 600, 700, 800, 900, or 1,000 mg of antibody. Liquid formulations can also be prepared so that any dosage may readily and conveniently be dispensed. In some embodiments, the antibody is administered the subject in an effective amount. An effective amount is generally 0.01 mg/kg to 500 mg/kg body weight per day. In some embodiments, the pharmaceutically acceptable compositions may be formulated so that a dosage of from 0.01 mg/kg to 200 mg/kg or from 0.01 mg/kg to 100 mg/kg body weight per day of the compound can be administered to a patient receiving these compositions (e.g., based on a 75 kg human, a dosage of from 0.75 mg to 7.5 g or 15 g). In certain embodiments, the compositions of the present invention pre-formulated to provide a dosage of from0.01 mg/kg to 70 mg/kg (e.g., based on a 75 kg human, a dosage of from 0.75 mg to 5.25 g). An effective amount of the antibody may be, for example, 0.05 mg/kg, 0.1 mg/kg, 1 mg/kg, 2 mg/kg, 3mg/kg, 4 mg/kg, 5 mg/kg, 6 mg/kg, 7 mg/kg or 8 mg/kg per dose (e.g., based on a 75 kg human, a dosage of from 3.75 mg to 600 mg). The dosage of the antibody may be administered once, twice, three times, four times, five times or more per week, once every week, once every two weeks, or even once every three weeks during the course of treatment. The timing of the dosing may be daily, once every two days, once every three days, once every four days, once every five days, weekly, once every two weeks or once every three weeks. Formulations comprising the antibody may be prepared so that any dosage may readily and conveniently be dispensed.

The term "concomitantly administering" means administering one or more therapeutic agents substantially concurrently. The term "concomitantly administering" encompasses not only administering two agents in a single pharmaceutical dosage form but also administration of each active agent in its own separate pharmaceutical dosage formulation. Where separate dosage formulations are used, the agents can be administered at essentially the same time, i.e., concurrently.

The term "sequentially administering" means administering agents at separately staggered times. Thus, for example, agents can be sequentially administered such that the beneficial pharmaceutical effect of aspirin and a compound of the present invention are realized by the patient at substantially the same time. Thus, for example, if a compound of the present invention and aspirin are both administered on a once a day basis, the interval of separation between sequential administration of the two agents can be up to twelve hours apart.

"Effective amount" or "treatment-effective amount" refers to amount that is effective for treating a cancer as noted through clinical testing and evaluation, patient observation, and/or the like. An "effective amount" can further designate an amount that causes a detectable change in biological or chemical activity. The detectable changes may be detected and/or further quantified by one skilled in the art for the relevant mechanism or process. Moreover, an "effective amount" can designate an amount that maintains a desired physiological state, i.e., reduces or prevents significant decline and/or promotes improvement in the condition. An "effective amount" can further refer to a therapeutically effective amount. "Subject" as used herein refers a mammalian subject, and particularly a human subject, including a male or female subject, and including a neonatal, infant, juvenile, adolescent, adult or geriatric subject, and further is inclusive of various races and ethnicities.

The terms "antibody" and "antibodies" as used herein is inclusive of all types of immunoglobulins, including IgG, IgM, IgA, IgD, and IgE, or fragments thereof, that may be appropriate for the medical uses disclosed herein. The antibodies may be monoclonal or polyclonal and may be of any species of origin, including, for example, mouse, rat, rabbit, horse, or human. Antibody fragments that retain specific binding to the protein or epitope, for example, CTLA4, PDL1 or PD1, bound by the antibody used in the present invention are included within the scope of the term "antibody." Such fragments can be produced by known techniques. The antibodies may be chimeric or humanized, particularly when they are used for therapeutic purposes. The antibody may be obtained or prepared using methods known in the art. "Antibody therapy" refers to the medical use of antibodies that bind a target cell or protein to treat cancer and/or stimulate an immune response in a subject that results in the recognition, attack and/or destruction of cancerous cells in the subject, and in some embodiments of the invention, to activate or stimulate a memory immune response in a subject that results in the subsequent recognition, attack and/or destruction of cancerous cells in the subject. "CTLA4 antibody therapy" refers to the use of antibodies directed toward cytotoxic t-lymphocyte antigen 4 (anti-CTLA4) in modulating an immune response in a subject. In some embodiments, the CTLA4 antibody inhibits or blocks the action of CTLA4 signaling that results in the inhibition of T-cell activation in the attack and destruction of cancer cells. Suitable antibodies for this use include, but are not limited to, antibodies that are CTLA4 antagonists or the CTLA4 antibodies as set forth in U.S. Patent Nos. 8,685,394 and 8,709,417. Some embodiments of the antibody include MDX-0l0 (ipilimumab, Bristol-Myers Squibb) and CP-675,206 (tremelimumab, Pfizer). In a particular embodiment, the antibody is ipilimumab. "PDL1 antibody therapy" refers to the use of antibodies directed toward programmed death ligand 1 (anti-PDL1) in modulating an immune response in a subject. In some embodiments, the PDL1 antibody inhibits or blocks the interaction of PDL1 with programmed cell death protein 1 (PD1), wherein the blockage of the interaction between PDL1 and PD1 inhibits the negative regulation of T -cell activation by PD 1 to attack and destroy cancer cells. Suitable antibodies for this use include, but are not limited to, the antibodies set

forth in U.S. Patent Nos. 8,217,149, 8,383,796, 8,552,154 and 8,617,546. In a particular embodiment, the antibody is MPDL3280A (Roche). "PD 1 antibody therapy" refers to the use of antibodies directed toward programmed cell death protein 1 PD1 (anti-PD1) in modulating an immune response in a subject. In some embodiments, the PD1 antibody inhibits or blocks the interaction of PD1 with PDL1, wherein the inhibition or blockage of the interaction between PDL1 and PD1 inhibits the negative regulation of T-cell activation by PD 1 to attack and destroy cancer cells. Suitable antibodies for this use include, but are not limited to, the antibodies set forth in U.S. Patent Nos. 7,029,674, 7,488,802, 7,521,051, 8,008,449, 8,354,509, 8,617,546 and 8,709,417. Particular embodiments of the antibody include nivolurnab (Bristol-Myers Squibb), labrolizurnab (Merck), and pernbrolizurnab (KEYTRUDA, Merck).

"Anti-metabolite chemotherapy" refers to the use of an anti-metabolite chemotherapeutic in the treatment of a subject. "Anti-metabolite" refers to a group of molecules that impede DNA and RNA synthesis. Examples of anti-metabolites include, but are not limited to, anti-folates, fluoropyrimidines, deoxynucleoside analogues and thiopurines. Anti-folates include methotrexate and pemetrexed. Fluoropyrirnidines include fluorouracil and capecitabine. Deoxynucleoside analogues include cytarabine, gemcitabine, decitabine, 5'-azacytidine (VIDAZA), fludarabine, nelarabine, cladribine, clofarabine and pentostatin. Thiopurines include thioguanine and mercaptopurine. In one embodiment, the anti-metabolite is gemcitabine. In another embodiment, the anti-metabolite is capecitebine. In order that the invention described herein may be more fully understood, the following examples are set forth. It should be understood that these examples are for illustrative purposes only and are not to be construed as limiting this invention in any manner.

## EXAMPLES

**Example 1.** *In vivo* pharmacodynamic test on the growth of colon cancer CT26 transplanted tumor in mice

### 1.1 Cell Culture

CT26 tumor cell line was maintained in vitro as monolayer culture in RPMI-1640 Media supplemented with 10% heat inactivated fetal calf serum at 37 °C in an atmosphere of 5% $CO_2$ in air. The tumor cells were routinely sub-cultured twice weekly by trypsin-EDTA treatment, not to exceed 4-5 passages. The cells growing in an exponential growth phase were harvested and counted for tumor inoculation.

### 1.2 Method for Tumor Inoculation

Each mouse was inoculated subcutaneously on the right lower flank with the single cell suspension of 95% viable tumor cells (3 x $10^5$) in 0.1 mL of serum-free RPMI 1640 Media for tumor development. Each mouse was administered with a test compound of this invention when mean tumor size reached approximately 100 $mm^3$. Each group consisted of 6 mice. The mice were randomized to different treatment groups as shown in the table under 'Groups and Treatment'. The mice were lightly anesthetized before implantation. Care was taken to ensure subcutaneous delivery of cells by lifting up the fold of skin with sterilized forceps and injecting cells. Any tumors that completely or partially grew intradermally (ID) or intramuscularly (IM) were not used.

### 1.3 In vivo Anti-tumor Pharmacology

CT26 cells were maintained in RPMI 1640 medium supplemented with 10% FBS at 37 °C and 5% CO2 atmosphere. Cell detachment was obtained using standard trypsinization methods, quantification of cell numbers and viability information using the NC-200 automated cell counter. Compound 1 (INV-1121) was thoroughly suspended in 0.5% methyl cellulose (MC) by sonication at 4 °C for 15 min before oral administration (p.o.) to animals. BALB/c mice were injected subcutaneously (s.c.) with live 1 X $10^5$ 4T1 cells. Mice developed tumors of approximately 36 $mm^3$ in 5 days. CT26 tumor-bearing mice were randomized and mapped into 5 groups of 10 mice each: group A received vehicle (0.5% MC); group B received 0.1 mg/kg of Compound 1; group C received 1 mg/kg of Compound 1, group D received 25 mg/kg of Compound 1; and group E received 150 mg/kg of Compound 1. All treatments were give Compound 1 p.o. daily for 21 consecutive days. Tumor volumes and body weights were measured twice weekly. Study was terminated 27 days after tumor cell injection. Tumor volumes were expressed as mean: t SEM. Tumor volume differences among treated mouse groups on day 27 were analyzed by the one-way ANOVA followed by Tukey's test. P <0.05 values were considered significant.

### 1.4 Measurement Parameters

For routine monitoring, all study animals were monitored not only tumor growth but also behavior such as mobility, food and water consumption (by cage side checking only), body weight (BW), eye/hair matting and any other abnormal

effect. All mortality and/or abnormal clinical signs were recorded.

1.4.1 Body Weight

Body weights of all animals are measured twice/week throughout the study. The measurement dates will specify as study design. Body weight change, expressed in %, was calculated using the following formula:

$$\text{BW change (\%)} = ((\text{BWDay X} - \text{BWDay 0})/\text{BWDay 0}) \times 100$$

1.4.2 Tumor Measurements

The measurement of tumor size was conducted twice/week with a caliper and the tumor volume (mm3) will be estimated using the formula: TV=a × b 2/2, where "a" and "b" is long and short diameters of a tumor, respectively. The TVs were used for calculation of the tumor growth inhibition (TGI, an indicator of antitumor effectiveness) value using the formula: TGI = (1-T/C) × 100%, where "T" and "C" is the mean relative volumes (% tumor growth) of the tumors in the treated and the control groups, respectively. The experiment was terminated when the mean tumor volume exceeded 2000 mm3 or severe body weight loss.

1.5 *In vivo* anti-tumor Activities

As described above, Compound 1 (INV-1121) was examined for its activity in tumor growth using a mouse colon CT26 syngeneic tumor model. Daily oral administration of the compound inhibited the tumor growth in a dose-dependent manner in general in the range of 0.1 mg/kg - 150 mg/kg (FIG. 1). At both lower doses (0.1 and 1.0 mg/kg) tested, some inhibition was observed, but without statistical significance. On the other hand, significant and comparable anti-tumor activity was detected for doses at 25 and 150 Mg/kg, indicating an optimal efficacious dose was reached by 25 mg/kg in vivo experimental setting. None of the doses tested showed gross toxicity judged from the animal body weight and overall animal behavior (FIG. 1), indicating an excellent tolerability in vivo tested animal species. The inhibitory effect of Compound 1 on tumor growth is shown in Figure 1

**Example 2:** *In vivo* pharmacodynamic test on the growth of colon cancer of subcutaneous Murine Colon Adenocarcinoma (MC38)

All animal studies were reviewed and approved by the Animal Care and Use Committee of Beth Israel Deaconess Medical Center, Boston, Massachusetts. Animals were housed at a maximum of 5 animals per cage in a pathogen-free facility with unlimited access to sterile water and chow. Daily welfare evaluations and animal sacrifices were carried out according to the Committee guidelines. MC38 mouse colon adenocarcinoma cells (Kerafast, Boston, MA, USA) were cultured in DMEM that was supplemented with 10% FBS, 1% GPS, 0.1 mM nonessential amino acids (MilliporeSigma), 1 mM sodium pyruvate (MilliporeSigma), 10 mM Hepes (MilliporeSigma), and 50 mg/mL gentamycin sulfate (MilliporeSigma). Adherent cells were trypsinized, pelleted, counted by hemocytometer, and injected into mice at $1x10^6$ cells/mL in PBS.

Cells were injected subcutaneously into the mid-dorsum of 6-week-old male C57BL/6 mice (The Jackson Laboratory, Bar Harbor, ME) at 100 µL/mouse. Mice were systemically treated with Compound 1 (INV-1120, 60 mg/kg/day), anti-PD1 (200 ug Q 3 days), INV-1120 (60 mg/kg/day) and anti-PD1 (200 ug Q 3 days), or vehicle (0.45% methylcellulose) in a total volume of 100 µL via orally by gavage. Treatment initiated on day 10 post-tumor cell injection, when tumors reached approximately 2004 $mm^3$ to 224 $mm^3$. Tumor size was measured by caliper ($width^2$ x length x 0.52 = $mm^3$).

After 15 days of treatment, 60 mg/kg/day INV-1120 (n=5 mice) inhibited primary MC38 murine colon adenocarcinoma (p<0.0001) vs control (n=5 mice).

After 21 days of treatment, anti-PD1 (200 ug Q3 days) (n=5 mice) inhibited primary MC38 murine colon adenocarcinoma (p<0.01) vs control (n=5 mice).

After 21 days of treatment, 60 mg/kg/day INV-1120 and anti-PD1 (200 ug Q 3 days) (n=5 mice) inhibited MC38 primary murine colon adenocarcinoma (p<0.00001) vs control (n=5 mice).

The inhibitory effects of the different treatments with or without Compound 1 on tumor growth are shown in Figure 2

**Example 3:** *In vivo* pharmacodynamic test on the growth of B16F10 melanoma (B16F10)

B16F10 ($1x10^6$ cells) were injected subcutaneously into the mid-dorsum of 6-week-old male C57BL/6 mice (The Jackson Laboratory, Bar Harbor, ME) at 100 μL/mouse. Mice were systemically treated with Compound 1 (INV-1120, 90 mg/kg/day), anti-PD1 (200 ug Q3 days), INV-1120 (90 mg/kg/day) and anti-PD1 (200 ug Q 3 days), or vehicle (0.45% methylcellulose) in a total volume of 100 μL via orally by gavage. Treatment initiated on day 10 post-tumor cell injection, when tumors reached approximately 100 $mm^3$ to 116 $mm^3$. Tumor size was measured by caliper ($width^2$ x length x 0.52 = $mm^3$).

After 8 days of treatment, 90 mg/kg/day INV-1120 (n=5 mice) inhibited primary 16F10 melanoma growth($p<0.001$) vs control (n=5 mice).

After 8 days of treatment, anti-PD1 (200 ug Q 3 days) inhibited primary 16F10 melanoma growth (n=5 mice) ($p<0.01$) vs control (n=5 mice).

After 8 days of treatment, 90 mg/kg/day INV-1120 and anti-PD1 (200 ug Q 3 days) (n=5 mice) inhibited primary 16F10 melanoma growth ($p<0.0001$) vs control (n=5 mice).

**Example 4:** *In vivo* pharmacodynamic test on the growth of LLC (Lewis Lung Cancer)

LLC ($1x10^6$ cells) were injected subcutaneously into the mid-dorsum of 6-week-old male C57BL/6 mice (The Jackson Laboratory, Bar Harbor, ME) at 100 μL/mouse. Mice were systemically treated with INV-1120 (90 mg/kg/day), anti-PD1 (200 ug Q 3 days), INV-1120 (90 mg/kg/day) and anti-PD1 (200 ug Q 3 days), or vehicle (0.45% methylcellulose) in a total volume of 100 μL via orally by gavage. Treatment initiated when tumors reached approximately 249 $mm^3$ to 297 $mm^3$. Tumor size was measured by caliper ($width^2$ x length x 0.52 = $mm^3$).

After 9 days of treatment, 90 mg/kg/day INV-1120 (n=5 mice) inhibited primary Lewis lung carcinoma growth ($p<0.01$) vs control (n=5 mice).

After 9 days of treatment, anti-PD1 (200 ug Q 3 days), (n=5 mice) inhibited primary Lewis lung carcinoma growth; $p= 0.056$ vs control (n=5 mice).

After 9 days of treatment, 90 mg/kg/day INV-1120 and anti-PD1 (200 ug Q 3 days) (n=5 mice) inhibited primary Lewis lung carcinoma growth($p<0.01$) vs control (n=5 mice).

Fig. 4 shows the in vivo inhibitory effect on the growth of Lewis Lung Cancer.

The data discussed above in Examples 1-4 and shown in Figs. 1-4 provide evidence that the heterocyclic amide EP4 antagonist of this invention had significant anti-tumor growth activity in various immunocompetent animal cancer models. Combination treatment of heterocyclic amide EP4 antagonist plus monoclonal antibody significantly enhanced the anti-tumor activity compared with treatment with antibody alone, and thus can have therapeutic use in the clinic for treating cancer.

**Example 5.** *In vivo* Anti-inflammatory Activities

Animal study was conducted to determine the activities of salts of Formula (I) against arthritis.

**Preparation of Animals**

Male Lewis Rates of 8-10 weeks old were fed with fixed amount of foods and free water at 20±2 °C, under light in the 12-hour light and 12-hour darkness cycle and with the humidity of 45-70%. All mice were provided adjusted living for three days before they were used in test. 56 of such mice were divided into 7 groups with 8 mice in each group. One group was a control group and received only 1% CMCNa solution; one group was a model group and also received only 1% CMCNa solution; one group was a positive group and received Celecoxib at 18 mg/kg, and the other four groups received solutions of tested compound at the 1 mg/kg, 3 mg/kg, 10 mg/kg and 40 mg/kg dosages respectively.

**Preparation of Formulations**

1% CMC-Na was prepared with 1.00277 g of CMC-Na in 100 mL distilled water, heated with a 60 °C water bath until CMC-Na fully dissolved.

18 mg/kg Celecoxib capsule: 18.09 mg Celecoxib was placed into a grinder into which 10 mL 1% CMC-Na solution was added slowly. The mixture was ground until Celecoxib fully dissolved.

10 mg/kg C-003 solution: 10.08 mg of a tested compound (4-(1-{[2-Methyl-4-(4-trifluoromethyl-benzyl)-4H-thieno[3,2-b]pyrrole-3-carbonyl]-amino}-cyclopropyl)-benzoic acid diethanolamino salt, which is referred to hereinafter as INV-1120 diethanolamino salt) was added into a grinder, into which 10 mL 1%CMC-Na solution was slowly added until INV-1120 diethanolamino salt fully dissolved.

30 mg/kg INV-1120 solution: 30.3 mg of INV-1120 diethanolamino salt was added into a grinder, into which 10 mL 1%CMC-Na solution was slowly added until INV-1120 diethanolamino salt fully dissolved.

1 mg/kg INV-1120 was prepared by mixing 1.0 mL of 10mg/kg INV-1120 diethanolamino salt solution with 9 mL 1% CMC-Na.

3 mg/kg INV-1120 was prepared by mixing 1.0 mL of 30mg/kg INV-1120 diethanolamino salt solution with 9 mL 1% CMC-Na.

**Establishment of the AIA Model**

After the mice were subject to Anesthesia, they were cleaned with medical alcohol at their right feet. The mice in the control group were administered with 50 uL PBS, and the mice of the other groups were treated at the right feet with 50 uL CDA solution. One day before the establishment of the model, the basic size (volume) of each mice was measured, the date of the model establishment was D1, test compound was administered at 1 mL/100g to the stomach starting on D13 daily for 12 days (ending on D24).

**Regular Examination of Animals**

The mice were examined and photos were taken every three days for their water intakes, food intakes, and weights; every four days for their abilities to withstand weight at their rear feet; every three days for the swelling volumes and swelling thickness and temperatures of their feet, as well as their behaviors.

The test results, as depicted in Figs. 5-6, show that the tested compound had effect in lowering the temperatures and swelling (both volumes and thickness) of the feet of the arthritis mice. In addition, mice administered with tested compounds showed greater weight gains and ability to withstand weight than those without administration of tested compounds. Further, the tested animals showed improved behavior patterns (e.g., balancing capability).

**Example 6.** *In vivo* Anti-inflammatory Activities

4-(1-{[2-Methyl-4-(4-trifluoromethyl-benzyl)-4H-thieno[3,2-b]pyrrole-3-carbonyl]-amino}-cyclopropyl)-benzoic acid tris(hydroxymethyl)aminomethane salt was also used in studies as described above for its activities in treating arthritis in mice. This salt resulted in even more effective reduction of swelling on the arthritis feet of the mice and better balancing or coordination ability of the mice after treatment with this compound.

The invention is defined by the appended claims.

## Claims

1. A compound for use in a method for treating a cancer or an inflammatory disease, the use comprising administering to a subject in need thereof a compound as below or a pharmaceutically acceptable salt thereof,

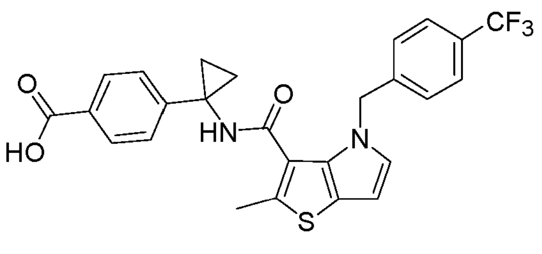

**INV-1120** or

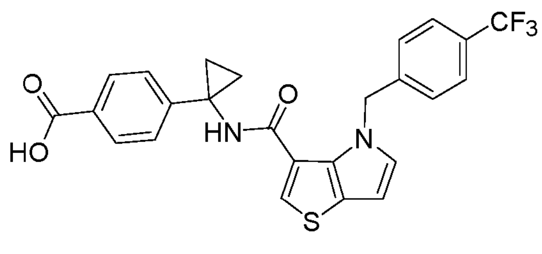

**INV-1121** .

2. The compound for use of claim 1, wherein the pharmaceutically acceptable salt is a diethanolamino salt or a tris(hydroxymethyl)aminomethane salt.

3. The compound for use of claim 1, wherein the cancer is breast cancer, endometrial cancer, cervix cancer, ovary cancer, lung cancer, head and neck cancer, brain cancer, thyroid cancer, esophagus cancer, stomach cancer, colon and rectal cancer, liver cancer, pancreatic cancer, skin cancer, kidney cancer, bladder cancer, prostate cancer, testis cancer, bone cancer, Lymphoma, or blood cancer.

4. The compound for use of claim 1, wherein the inflammatory disease is arthritis, acne vulgaris, asthma, autoimmune diseases, autoinflammatory diseases, Celiac disease, chronic prostatitis, colitis, diverticulitis, glomerulonephritis, hidradenitis suppurativa, hypersensitivities, inflammatory bowel diseases, interstitial cystitis, Mast Cell Activation Syndrome, mastocytosis, otitis, pelvic inflammatory disease, reperfusion injury, rheumatic fever, rheumatoid arthritis, rhinitis, sarcoidosis, or vasculitis.

5. The compound for use of claim 1, wherein the use further comprises a radiation therapy, an anti-metabolite chemotherapy, or an antibody therapy, wherein the antibody is selected from antibodies to cytotoxic t-lymphocyte antigen 4 (anti-CTLA4), antibodies to programmed death ligand 1 (anti-PDL1), and antibodies to programmed cell death protein 1 (anti-PD1).

6. A pharmaceutical composition for use in the treatment of cancer or an inflammatory disease, comprising a compound described in claim 1 or 2, and a pharmaceutically acceptable carrier.

7. The pharmaceutical composition for use of claim 6, wherein the composition further comprises an antimetabolite or an antibody; wherein the antibody is selected from antibodies to cytotoxic t-lymphocyte antigen 4 (anti-CTLA4), antibodies to programmed death ligand 1 (anti-PDL1), and antibodies to programmed cell death protein 1 (anti-PD1).

## Patentansprüche

1. Verbindung zur Verwendung in einem Verfahren zur Behandlung eines Krebses oder einer entzündlichen Erkrankung, wobei die Verwendung Verabreichen einer Verbindung wie nachstehend oder eines pharmazeutisch unbedenklichen Salzes davon an ein Subjekt umfasst, das dies benötigt,

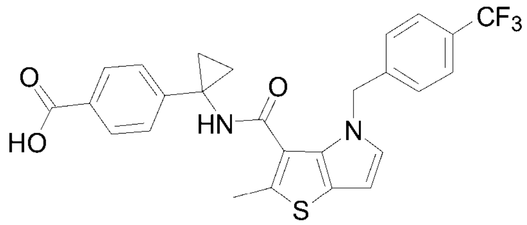

**INV-1120**

oder

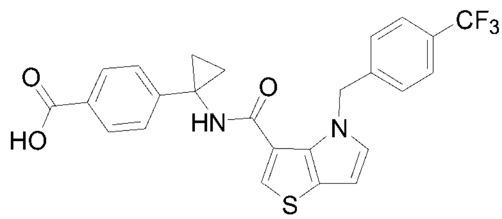

**INV-1121**

.

2. Verbindung zur Verwendung nach Anspruch 1, wobei es sich bei dem pharmazeutisch unbedenklichen Salz um ein Diethanolaminosalz oder ein Tris(hydroxymethyl)aminomethansalz handelt.

3. Verbindung zur Verwendung nach Anspruch 1, wobei es sich bei dem Krebs um Brustkrebs, Endometriumkarzinom, Gebärmutterhalskrebs, Eierstockkrebs, Lungenkrebs, Kopf-Hals-Krebs, Hirnkrebs, Schilddrüsenkrebs, Speiseröhrenkrebs, Magenkrebs, Dickdarm- und Rektumkrebs, Leberkrebs, Bauchspeicheldrüsenkrebs, Hautkrebs, Nierenkrebs, Blasenkrebs, Prostatakrebs, Hodenkrebs, Knochenkrebs, Lymphom oder Blutkrebs handelt.

4. Verbindung zur Verwendung nach Anspruch 1, wobei es sich bei der entzündlichen Erkrankung um Arthritis, Akne vulgaris, Asthma, Autoimmunerkrankungen, autoinflammatorische Erkrankungen, Zöliakie, chronische Prostatitis, Colitis, Divertikulitis, Glomerulonephritis, Hidradenitis suppurativa, Überempfindlichkeiten, entzündliche Darmerkrankungen, interstitielle Zystitis, Mastzellaktivierungssyndrom, Mastozytose, Otitis, entzündliche Beckenerkrankung, Reperfusionsschaden, rheumatisches Fieber, rheumatoide Arthritis, Rhinitis, Sarkoidose oder Vaskulitis handelt.

5. Verbindung zur Verwendung nach Anspruch 1, wobei die Verwendung ferner eine Strahlentherapie, eine Antimetabolit-Chemotherapie oder eine Antikörpertherapie umfasst, wobei der Antikörper ausgewählt ist aus Antikörpern gegen zytotoxisches T-Lymphozyten-Antigen 4 (Anti-CTLA4), Antikörpern gegen programmierten Zelltod-Liganden 1 (Anti-PDL1) und Antikörpern gegen programmiertes Zelltodprotein 1 (Anti-PD1).

6. Pharmazeutische Zusammensetzung zur Verwendung bei der Behandlung von Krebs oder einer entzündlichen Erkrankung, umfassend eine in Anspruch 1 oder 2 beschriebene Verbindung und einen pharmazeutisch unbedenklichen Träger.

7. Pharmazeutische Zusammensetzung zur Verwendung nach Anspruch 6, wobei die Zusammensetzung ferner einen Antimetaboliten oder einen Antikörper umfasst; wobei der Antikörper ausgewählt ist aus Antikörpern gegen zytotoxisches T-Lymphozyten-Antigen 4 (Anti-CTLA4), Antikörpern gegen programmierten Zelltod-Liganden 1 (Anti-PDL1) und Antikörpern gegen programmiertes Zelltodprotein 1 (Anti-PD1).

**Revendications**

1. Composé destiné à être utilisé dans un procédé de traitement d'un cancer ou d'une maladie inflammatoire, l'utilisation comprenant l'administration, à un sujet qui en a besoin, d'un composé tel que décrit ci-dessous ou d'un sel pharmaceutiquement acceptable de celui-ci,

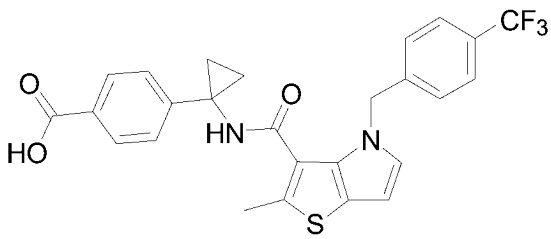

**INV-1120**

ou

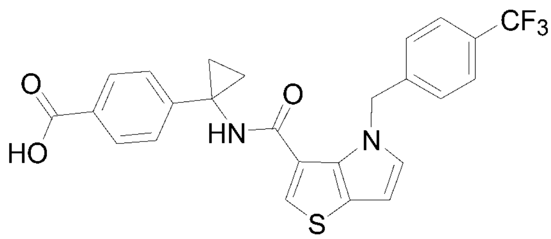

**INV-1121**

.

2. Composé destiné à être utilisé selon la revendication 1, dans lequel le sel pharmaceutiquement acceptable est un sel de diéthanolamino ou un sel de tris(hydroxyméthyl)aminométhane.

3. Composé destiné à être utilisé selon la revendication 1, dans lequel le cancer est un cancer du sein, un cancer de l'endomètre, un cancer du col de l'utérus, un cancer de l'ovaire, un cancer du poumon, un cancer de la tête et du cou, un cancer du cerveau, un cancer de la thyroïde, un cancer de l'œsophage, un cancer de l'estomac, un cancer du côlon et du rectum, un cancer du foie, un cancer du pancréas, un cancer de la peau, un cancer du rein, un cancer de la vessie, un cancer de la prostate, un cancer des testicules, un cancer des os, un lymphome ou un cancer du sang.

4. Composé destiné à être utilisé selon la revendication 1, dans lequel la maladie inflammatoire est l'arthrite, l'acné vulgaire, l'asthme, les maladies auto-immunes, les maladies auto-inflammatoires, la maladie cœliaque, la prostatite chronique, la colite, la diverticulite, la glomérulonéphrite, l'hidradénite suppurée, les hypersensibilités, les maladies inflammatoires de l'intestin, la cystite interstitielle, le syndrome d'activation mastocytaire, la mastocytose, l'otite, la maladie inflammatoire pelvienne, les lésions de reperfusion, le rhumatisme articulaire aigu, la polyarthrite rhumatoïde, la rhinite, la sarcoïdose ou la vascularite.

5. Composé destiné à être utilisé selon la revendication 1, dans lequel l'utilisation comprend en outre une radiothérapie, une chimiothérapie antimétabolite ou une thérapie par anticorps, dans lequel l'anticorps est choisi parmi les anticorps dirigés contre l'antigène 4 des lymphocytes T cytotoxiques (anti-CTLA4), les anticorps dirigés contre le ligand 1 de la mort programmée (anti-PDL1) et les anticorps dirigés contre la protéine 1 de la mort cellulaire programmée (anti-PD1).

6. Composition pharmaceutique destinée à être utilisée dans le traitement du cancer ou d'une maladie inflammatoire, comprenant un composé décrit dans la revendication 1 ou 2, et un support pharmaceutiquement acceptable.

7. Composition pharmaceutique destinée à être utilisée selon la revendication 6, dans laquelle la composition comprend en outre un antimétabolite ou un anticorps ; dans laquelle l'anticorps est choisi parmi les anticorps dirigés contre l'antigène 4 des lymphocytes T cytotoxiques (anti-CTLA4), les anticorps dirigés contre le ligand 1 de la mort programmée (anti-PDL1) et les anticorps dirigés contre la protéine 1 de la mort cellulaire programmée (anti-PD1).

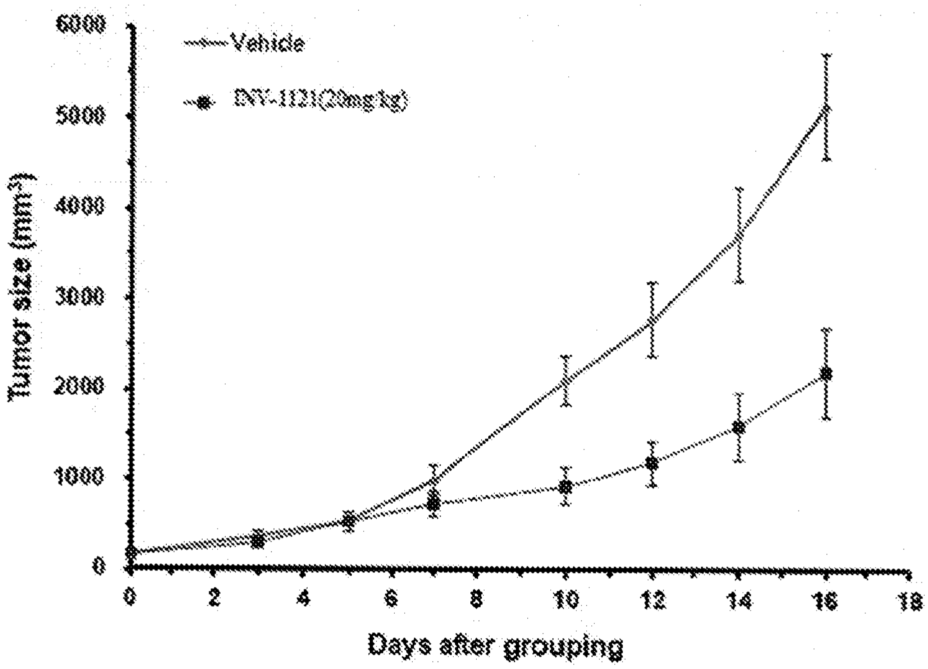
Vehicle
INV-1121(20mg/kg)
Tumor size (mm$^3$)
6000
5000
4000
3000
2000
1000
0
0
2
4
6
8
10
12
14
16
18
Days after grouping

Fig. 1

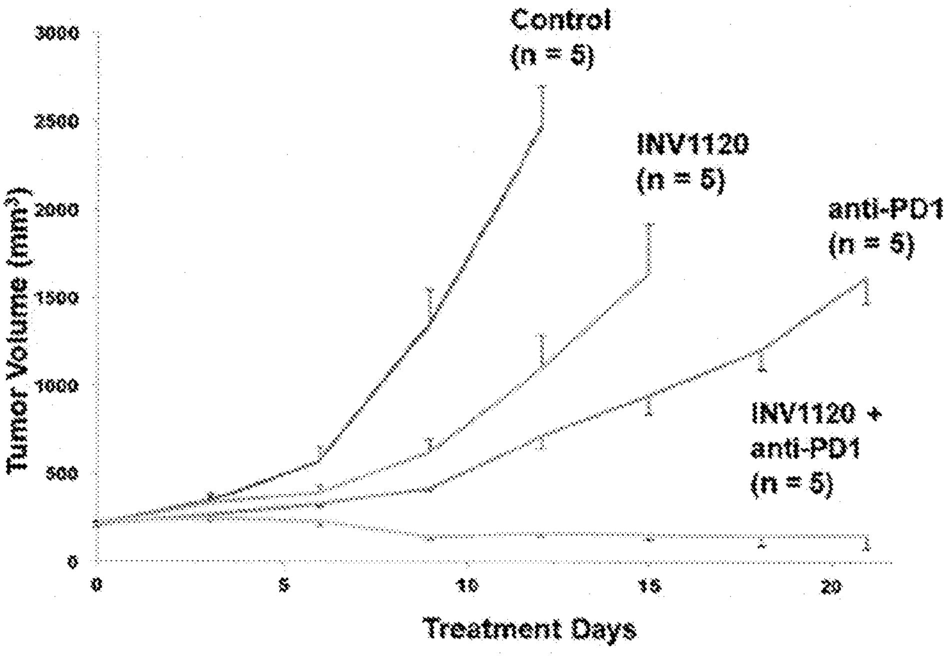
Control
(n = 5)
INV1120
(n = 5)
anti-PD1
(n = 5)
INV1120 +
anti-PD1
(n = 5)
Tumor Volume (mm$^3$)
3000
2500
2000
1500
1000
500
0
0
5
10
15
20
Treatment Days

Fig. 2

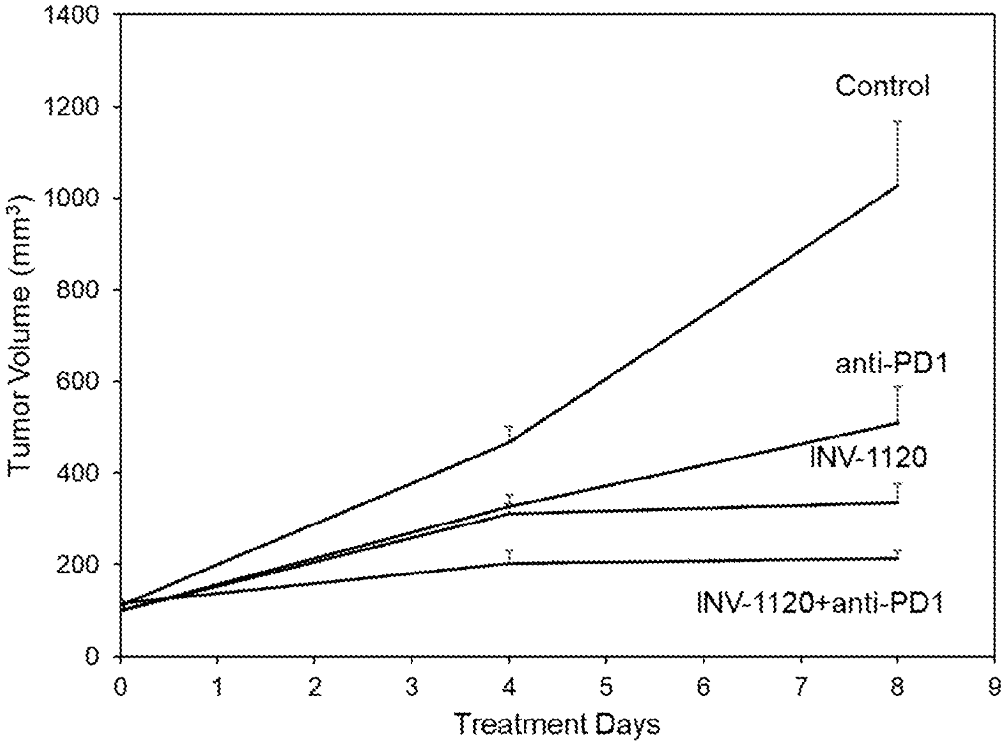
1400
1200
1000
800
600
400
200
0
Tumor Volume (mm3)
Control
anti-PD1
INV-1120
INV-1120+anti-PD1
0
1
2
3
4
5
6
7
8
9
Treatment Days

FIG. 3

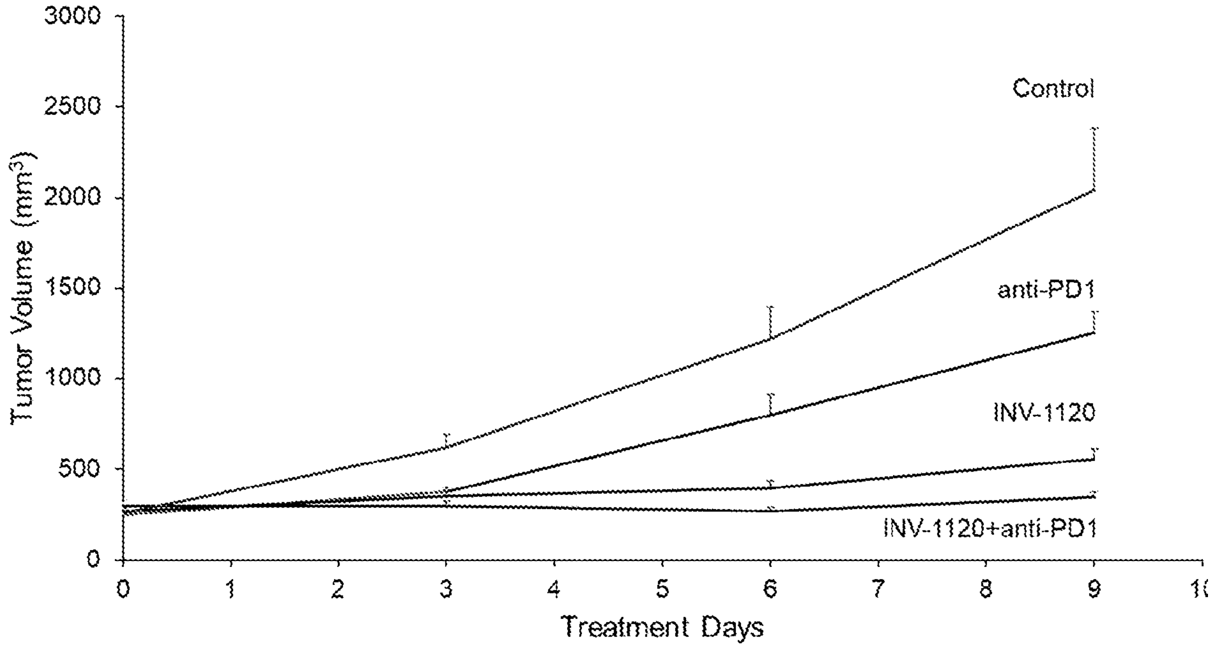
3000
2500
2000
1500
1000
500
0
Tumor Volume (mm3)
Control
anti-PD1
INV-1120
INV-1120+anti-PD1
0
1
2
3
4
5
6
7
8
9
Treatment Days

FIG. 4

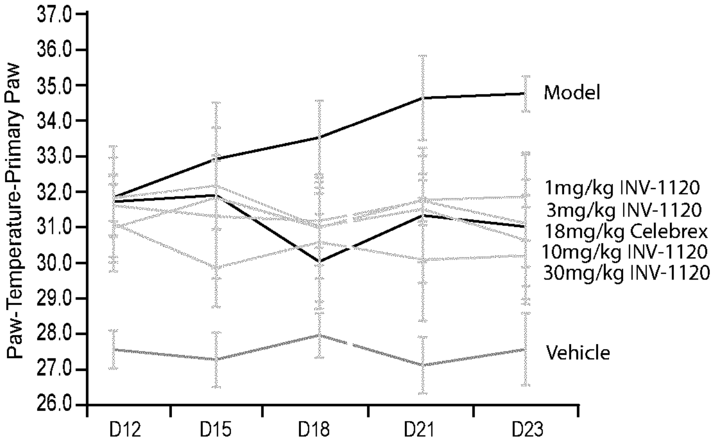

37.0
36.0
35.0
34.0
33.0
32.0
31.0
30.0
29.0
28.0
27.0
26.0
Paw-Temperature-Primary Paw
Model
1mg/kg INV-1120
3mg/kg INV-1120
18mg/kg Celebrex
10mg/kg INV-1120
30mg/kg INV-1120
Vehicle
D12
D15
D18
D21
D23


FIG.5

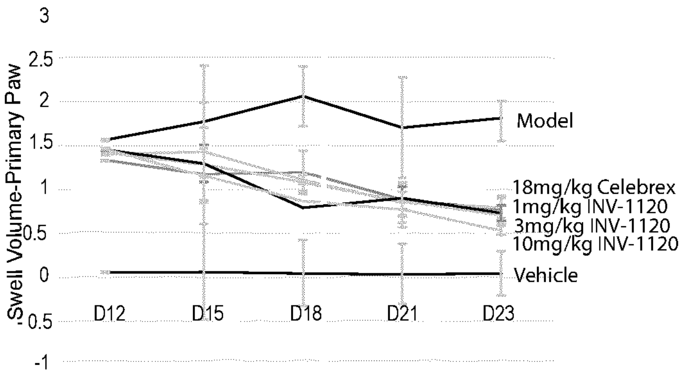

3
2.5
2
1.5
1
0.5
0
-0.5
-1
Swell Volume-Primary Paw
Model
18mg/kg Celebrex
1mg/kg INV-1120
3mg/kg INV-1120
10mg/kg INV-1120
Vehicle
D12
D15
D18
D21
D23


FIG.6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9606822 A
- WO 9611902 A
- EP 752421 A1
- WO 0316254 A
- WO 05021508 A
- WO 07121578 A
- US 20090537482 W
- WO 2010019796 A
- US 4256108 A
- US 4166452 A
- US 4265874 A
- US 8685394 B
- US 8709417 B
- US 8217149 B
- US 8383796 B
- US 8552154 B
- US 8617546 B
- US 7029674 B
- US 7488802 B
- US 7521051 B
- US 8008449 B
- US 8354509 B


### Non-patent literature cited in the description


- Eicosanoids: From Biotechnology to Therapeutic Applications,. Plenum Press, 1996, 137-154
- *Journal of Lipid Mediators and Cell Signaling*, 1996, vol. 14, 83-87
- *Prostaglandins and Other Lipid Mediators*, 2002, vol. 69, 557-573
- **WHITESIDE**. *Expert Opinion in Biological Therapy*, 2010, vol. 10, 1019-1035
- **YOKOYAMA U et al.** *Pharmacol. Rev*, 2013, vol. 65, 1010-1052
- **CHEN et al.** *British J Pharmacol.*, 2010, vol. 160, 292-310
- **MUTOH M et al.** *Cancer Res.*, 2002, vol. 62, 28-32
- **YANG et al.** *Cancer Res.*, 2006, vol. 66, 9665-9672
- **MAO Y et al.** *Clin. Cancer Res.*, 2014, vol. 20, 4096-4106
- Remington, The Science and Practice of Pharmacy. 1995